# EUROPEAN PATENT APPLICATION

(11) **EP 3 782 529 A1**
(43) Date of publication of application: **24.02.2021**
(21) Application number: 20191555.0
(22) Date of filing: 18.08.2020
(51) Int. Cl.: A61B 1/00, A61B 1/05, A61B 1/06

(54) **SYSTEMS AND METHODS FOR SELECTIVELY VARYING RESOLUTIONS**

(30) Priority: 19.08.2019 US 201962888654 P; 25.06.2020 US 202016912464
(71) Applicant: Covidien LP, Mansfield, MA 02048 (US)
(72) Inventor: KOMP, John W., Dillon, CO 80435 (US)
(74) Representative: Soames, Candida Jane

(57) **Abstract**

Methods and systems for generating a three-dimensional (3D) scan of a body inside of a patient include automatically interleaving scanning of a focused area within a first field of view (FOV) of an image sensor using a first light source in a fine mode with scanning of an area of the first FOV in a coarse mode to generate a scanned image of the body within the first FOV and generating 3D scan data of the body within the first FOV based on the scanned image.

## Description

### CROSS-REFERENCE TO RELATED APPLICATION

The present application claims the benefit of and priority to U.S. Provisional Patent Application Serial No. 62/888,654, filed on August 19, 2019, the entire content of which is hereby incorporated by reference herein.

### FIELD

The present technology is generally related to scanning systems and methods and, more particularly, to scanning systems and methods for generating a three-dimensional (3D) scan of a surgical site by selectively varying resolutions.

### BACKGROUND

Current monocular optical devices (e.g., endoscopes, bronchoscopes, colonoscopes) used for viewing surgical fields during minimally invasive surgery (e.g., laparoscopy) and visual diagnostic procedures (e.g., colonoscopy, bronchoscopy) provide limited reference information on anatomical features because the images obtained have no depth of field. To compensate, a surgeon may advance the surgical tool until it comes in contact with an aspect or another tool. This leads to inefficient motion. Binocular (also known as stereoscopic) optical devices provide limited depth of field affording the surgeon visual information on the distance between items within the optical device's field of view. The accuracy of distance information is limited based on the amount of parallax provided by the optical paths, determined by the distance between the optical paths, and the amount of overlap between the two optical paths.

During the course of a surgery, the area of interest may change due to, for example, a change in the position of surgical instrumentation, a change in the target anatomical feature, a change in the shape or structure of anatomical features, and/or for other reasons. A Surgeon needs to see such changes in real time, or as close to real time as possible, and with sufficient resolution to be able to accurately estimate the relative positioning of surgical instruments and anatomical features.

### SUMMARY

This disclosure generally relates to scanning systems and methods for generating 3D scan data of a surgical site. In particular, the systems and methods of the disclosure enable scanning at least a first portion of an area of interest in at least one fine mode and at least a second portion of the area of interest in at least one coarse mode such that the first portion of the area of interest may be displayed with a higher resolution than the second portion of the area of interest. In this manner, the systems and methods of this disclosure strike a balance between providing higher resolution and minimizing scan time.

Provided in accordance with embodiments of the disclosure is a method for generating a three-dimensional (3D) scan of a body inside of a patient. The method includes automatically interleaving scanning of a focused area within a first field of view (FOV) of an image sensor using a first light source in a fine mode with scanning of an area of the first FOV in a coarse mode to generate a scanned image of the body within the first FOV, and generating 3D scan data of the body within the first FOV based on the scanned image.

In an aspect of the disclosure, a distance between two consecutive scanning lines in the coarse mode is larger than a distance between two consecutive scanning lines in the fine mode.

In an aspect of the disclosure, a resolution outside of the focused area in the scanned image is lower than a resolution within the focused area in the scanned image.

In an aspect of the disclosure, scanning the focused area in the fine mode is performed during a predetermined time. A time to complete one scan line in the fine mode is determined by a length of the focused area. A speed of scanning in the fine mode is inversely proportional to a length of the focused area.

In an aspect of the disclosure, a ratio of an area scanned in the coarse mode to an area scanned in the fine mode is greater than or equal to 1.

In an aspect of the disclosure, the method further includes capturing a series of images of a portion of the body within a second FOV of an endoscope using a second light source. The method further includes calculating a difference between two of the series of images.

In another aspect of the disclosure, the focused area is received when the difference is greater than or equal to a predetermined threshold.

In still another aspect of the disclosure, the focused area includes an area, where the difference resides in majority.

In still another aspect of the disclosure, the second FOV of the endoscope is not less than the first FOV of the image sensor.

In yet another aspect of the disclosure, the method further includes receiving the focused area when an area overlapped by the second FOV and the first FOV is less than a predetermined area.

In still yet another aspect of the disclosure, the method further includes automatically designating the focused area in which the first FOV and the second FOV do not overlap.

A 3D scanner provided in accordance with embodiments of the disclosure includes an image sensor having a first field of view (FOV) using a first light source and configured to generate a series of images of a body inside of a patient, a scan image sensor having a second FOV using a second light source, and configured to scan an area of the body within the second FOV and generate a scanned image, and a processor configured to control the scan image sensor to scan the area of the body within the second FOV in a coarse mode and within a focused area in a fine mode and to generate 3D scan data of the body within the second FOV based on the series of images and the scanned image. The processor is further configured to control the scan image sensor to automatically interleave scanning the focused area in the fine mode with scanning the area within the second FOV in the coarse mode. The focused area is located within the area of the body.

In an aspect of the disclosure, the second light source emits infrared (IR) light.

In another aspect of the disclosure, the first light source emits visible light.

In another aspect of the disclosure, a distance between two consecutive scanning lines in the coarse mode is larger than a distance between two consecutive scanning lines in the fine mode.

In another aspect of the disclosure, a resolution outside of the focused area in the scanned image is lower than a resolution within the focused area in the scanned image.

In another aspect of the disclosure, scanning in the fine mode is performed during a predetermined time. The processor is further configured to determine a time to complete one scan line in the fine mode based on a length of the focused area. A speed of scanning in the fine mode is inversely proportional to a length of the focused area.

In still another aspect of the disclosure, the processor is further configured to calculate a difference between the series of images and the scanned image obtained in the coarse mode. The focused area is determined when the difference is greater than or equal to a predetermined threshold.

In yet another aspect of the disclosure, the first FOV of the image sensor is not less than the second FOV of the scan image sensor.

In yet still another aspect of the disclosure, the focused area is determined when an area overlapped by the second FOV and the first FOV is less than a predetermined area.

A method provided in accordance with embodiments of the disclosure is for imaging a body inside of a patient. The method includes receiving a three-dimensional (3D) model of the body, determining whether or not an area of a field of view (FOV) of a scan camera is contained in the 3D model, scanning the area of the FOV in a coarse mode when it is determined that the area of the FOV is contained in the 3D model, automatically interleaving scanning of a focused area within the FOV in a fine mode with scanning of the FOV in the coarse mode when it is determined that the area of the FOV is not contained in the 3D mode, generating a scanned image of the FOV by the scan camera, and generating an intra 3D model based on the 3D model and the scanned image by the image sensor.

The details of one or more embodiments of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various techniques are illustrated in the accompanying figures with the intent that these examples not be restrictive. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. The figures are listed below.
FIG. 1 is a schematic diagram of a scanning system for generating 3D scan data of a surgical site according to embodiments of the disclosure;
FIG. 2 is a perspective, partial cross-sectional view illustrating a scanning device of the scanning system of FIG. 1 in use inside a patient's body, according to embodiments of the disclosure;
FIG. 3 is an enlarged, perspective, partial view of the scanning device of FIG. 2, according to embodiments of the disclosure;
FIG. 4A is a block diagram illustrating patterns of coarse scanning of a surgical site according to embodiments of the disclosure;
FIG. 4B is a block diagram illustrating patterns of fine scanning of a surgical site according to embodiments of the disclosure;
FIG. 5 is a graphical illustration of 3D scan data having variable resolutions according to embodiments of the disclosure;
FIG. 6 is a block diagram of a computer device according to embodiments of the disclosure;
FIG. 7A is a flowchart for updating a 3D model according to an embodiment of the disclosure;
FIG. 7B is a flowchart for updating a 3D model according to another embodiment of the disclosure; and
FIG. 8 is a flowchart for generating 3D scan data of a surgical site according to embodiments of the disclosure.

### DETAILED DESCRIPTION

Visually displaying a target of interest within a surgical site is helpful for a surgeon to determine surgical instruments and/or anatomical features at the target. A three-dimensional (3D) image of the surgical site is further advantageous in that it provides the depth of field that is lacking in two-dimensional (2D) images. In order to provide 3D imaging, a scanner may be incorporated into an endoscope. The scanner scans the area of interest from which a 3D image is generated. The greater the desired resolution, the slower the scan speed. This disclosure provides systems and methods that strike a balance between providing higher resolution and minimizing scan time. More specifically, the systems and methods of this disclosure provide different resolutions in one scan image of the surgical site, thus providing detailed structural information of the surgical site of interest with a high resolution and general information thereof with a low resolution.

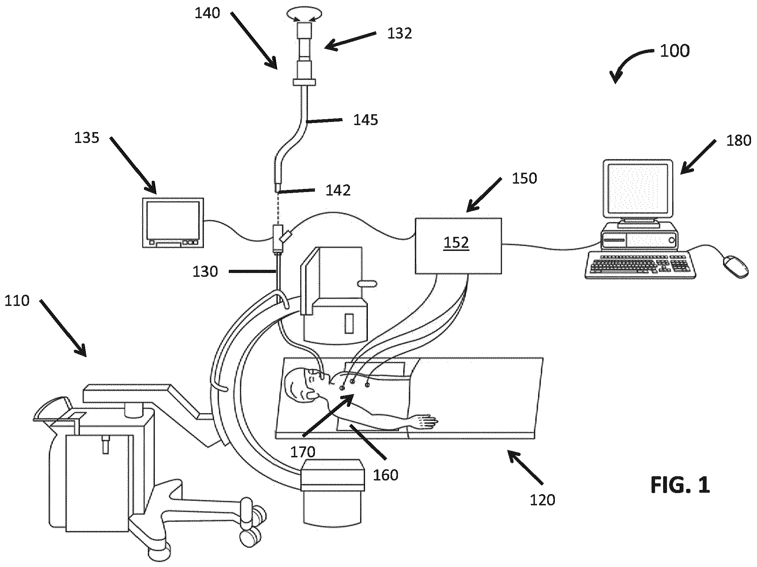

FIG. 1 illustrates a scanning system 100 for generating 3D volumetric data in accordance with embodiments of the disclosure. The scanning system 100 may be configured to construct 3D volumetric data around a target area including at least a portion of an organ of a patient from 2D medical images. The scanning system 100 may be further configured to advance a medical device to the target area and to determine the location of the medical device with respect to the target by using an electromagnetic navigation (EMN) system.

The scanning system 100 may be configured for reviewing 2D medical image data to identify one or more targets, planning a pathway to an identified target (planning phase), navigating an extended working channel (EWC) 145 of a catheter guide assembly 140 to a target (navigation phase) via a user interface, confirming placement of the EWC 145 relative to the target, and generating and displaying a 3D images of the scanned area. One such electromagnetic navigation system is the ELECTROMAGNETIC NAVIGATION BRONCHOSCOPY® system currently sold by Medtronic PLC. The target may be tissue of interest identified by reviewing the 2D medical image data during the planning phase. Following navigation, a medical device, such as a biopsy tool or other tool, may be inserted into the EWC 145 to obtain a tissue sample from the tissue located at or proximate to the target.

The EWC 145 is a part of the catheter guide assembly 140. In practice, the EWC 145 is inserted into an endoscope 130 for access to a target of interest inside the patient. The endoscope 130 may be any imaging device capable of navigating, capturing 2D images, or transmitting live view images of organs located within a patient. For example, the endoscope 130 is shown as a bronchoscope and may be a laparoscope.

The EWC 145 of the catheter guide assembly 140 may be inserted into a working channel of the endoscope 130 for navigation through a patient's inside body. A locatable guide (LG) 132, including a sensor 142, is inserted into the EWC 145 and locked into a position such that the sensor 142 extends a desired distance beyond the distal tip of the EWC 145. The position and orientation of the sensor 142 relative to the reference coordinate system, and thus the distal portion of the EWC 145, within an electromagnetic field can be derived. Such catheter guide assemblies 140 are currently marketed and sold by Medtronic PLC under the brand names SUPERDIMENSION® Procedure Kits, or EDGE™ Procedure Kits, and are contemplated as useable with the disclosure.

The scanning system 100 may include an operating table 120 configured to support the patient, the endoscope 130, monitoring equipment 135 (e.g., a video display for displaying video images) coupled to the endoscope 130, a locating system 150 including a locating module 152, a plurality of reference sensors 170, an electromagnetic wave transmitter mat 160, and a computing device 180 including software and/or hardware used to facilitate identification of a target, pathway planning to the target, navigation of a medical device to the target, confirmation of placement of the EWC 145 or a suitable device therethrough relative to the target, and generation of 3D scan data of the target or any organ of interest.

A medical imaging device 110 may be capable of acquiring fluoroscopic or x-ray images or video of the patient is also included in the scanning system 100. The images, sequence of images, or video captured by the medical imaging device 110 may be stored within the medical imaging device 110 or transmitted to computing device 180 for storage, processing, and display. Additionally, the medical imaging device 110 may move relative to the patient so that images may be acquired from different angles or perspectives relative to patient to create a sequence of fluoroscopic or x-ray images such as a video. The pose of the medical imaging device 110 relative to patient and for the images may be estimated via the structure of markers implanted in or placed around the patient. Structure of markers may be coupled to the transmitter mat (both indicated 160) and positioned under the patient on the operating table 120. Structure of markers and transmitter mat 160 may be two separate elements which may be coupled in a fixed manner or alternatively may be manufactured as one unit. The medical imaging device 110 may include a single imaging device or more than one imaging device. In case when multiple imaging devices are included, each imaging device may be a different or same type from each other.

The computing device 185 may be any suitable computing device including a processor and a storage medium, wherein the processor is capable of executing instructions stored on the storage medium. The computing device 185 may further include a database configured to store patient data, computed tomography (CT) data sets including CT images, further image data sets including fluoroscopic or x-ray images and video, navigation plans, 3D scan data, and any other medical image data. Although not explicitly illustrated, the computing device 185 may include inputs, or may otherwise be configured to receive, CT data sets, fluoroscopic or x-ray images/video and other data described herein. Additionally, the computing device 185 may include a display configured to display graphical user interfaces. The computing device 185 may be connected to one or more networks through which one or more databases may be accessed.

With respect to the planning phase, the computing device 185 utilizes previously acquired CT image data for generating and viewing a 3D model of the patient's body (e.g., lung), enables the identification of a target of interest on the 3D model, and allows for determining a pathway to tissue located at and around the target. More specifically, CT images acquired from previous CT scans are processed and assembled into a 3D CT volume, which is then utilized to generate a 3D model of the patient's body. The 3D model may be displayed on a display associated with the computing device 185, or in any other suitable fashion. Using the computing device 185, various views of the 3D model or enhanced 2D images generated from the 3D model are presented. The enhanced 2D images may possess some 3D capabilities because they are generated from 3D data. The 3D model may be manipulated to facilitate identification of a target on the 3D model or 2D images, and selection of a suitable pathway through the patient's airways to access tissue located at the target can be made. Once selected, the pathway plan, 3D model, and images derived therefrom, can be saved and exported to a navigation system for use during the navigation phase(s). One such planning software is the ILOGIC® planning suite currently sold by Medtronic PLC.

With respect to the navigation phase, a six degrees-of-freedom electromagnetic locating or tracking system 150 is utilized for performing registration of the images and the pathway for navigation, although other configurations are also contemplated. The tracking system 150 may include a locating or tracking module 152, a plurality of reference sensors 170, and the transmitter mat 160. The tracking system 150 is configured for use with the LG 132 and particularly the sensor 142. As described above, the LG 132 and the sensor 142 are configured for insertion through the EWC 145 into the patient's body and may be selectively lockable relative to one another via a locking mechanism.

The transmitter mat 160 generates an electromagnetic field around at least a portion of the patient within which the position of a plurality of reference sensors 170 and the sensor 142 can be determined with use of the tracking module 152. One or more of reference sensors 170 are attached to the chest of the patient. The six degrees of freedom coordinates of the reference sensors 170 are sent to the computing device 180 (which includes the appropriate software) where they are used to calculate a patient coordinate frame of reference. Registration is generally performed to coordinate locations of the 3D model and 2D images from the planning phase with respect to the patient as observed through the endoscope 130, and allow for the navigation phase to be undertaken with precise knowledge of the location of the sensor 142, even in portions of the airway where the endoscope 130 cannot reach. Further details of such a registration technique and their implementation can be found in U.S. Patent Application Pub. No. 2011/0085710, the entire content of which is incorporated herein by reference, although other suitable techniques are also contemplated.

Though the above-described EMN system is useable for endobronchial navigation within the lungs the systems and methods of the disclosure are not so limited. For example, it is contemplated that the devices herein may be utilized for other organs within a patient's body, such as the liver, kidneys, etc., and may be useable for scanning and visualizing organs during abdominal, video assisted thoracoscopic surgery, robot assisted thoracic surgery, and other procedures where scanning a FOV with structured light and supplementing the image with additional details may be employed.

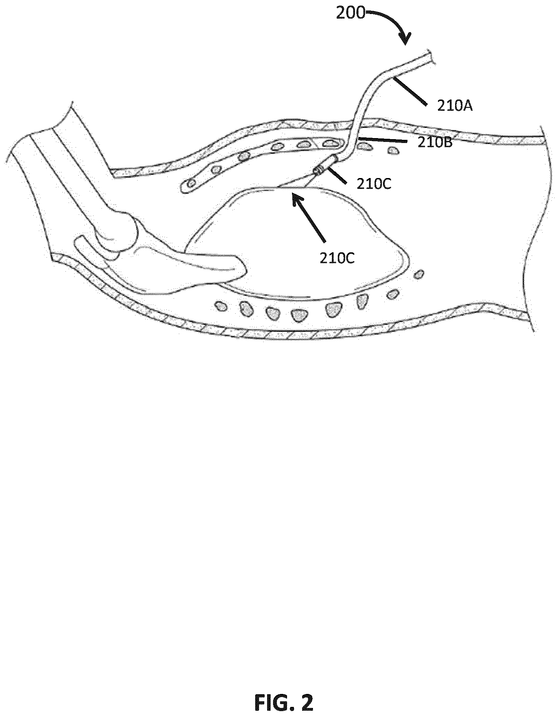

In case of resection or dissection of an internal organ, the endoscope may be inserted through an orifice or opening of the patient's body to navigate to the target. For performing a laparoscopic surgery, a surgical device may enter into the patient's body through another opening. FIG. 2 illustrates a side, cross-sectional, view of a thoracic cavity of a patient with an endoscope 200 having surface scanning capabilities disposed partially therein. The endoscope 200 is equipped with a scanner to display information of an internal organ, such as a liver, prior to, during, and after diagnosis and/or surgery, according to embodiments of the disclosure. A 3D map of a surface of a surgical site (e.g., a 3D model) may be generated by the computing device 180 of FIG. 1 or may be generated by using the endoscope 200 including a scanner, which draws a pattern across the surface of the surgical site (e.g., infrared projections), while capturing images of the surgical site (including the scanned surface) to generate 3D scan data. For example, the 3D scan data may be generated by analyzing the distortion of the images from reflections of projections projected by the scanner. The distortions in the captured images can be used to extract depth information to create the 3D scan data. By increasing or reducing the number of scanning lines, the scanner of the endoscope 200 may be able to generate detailed information of the portion of interest.

While description of the endoscope 200 with respect to the environment illustrated in FIG. 1 and 2 refer to use of the endoscope 200 without assistance of a trocar or other such delivery system, the endoscope 200 may be configured to be extended through the trocar or other such delivery system. Further, the endoscope 200 may be extended through a natural orifice or surgically created opening. The endoscope 200 includes an elongated body 210 configured to advance within a suitable trocar or other delivery device capable of receiving and subsequently delivering the endoscope 200 or other medical devices (e.g., an endobronchial catheter, thoracic catheter, trocar, and the like) into the body. The elongated body 210 may include first, second, and third segments 210A, 210B, 210C, each coupled to each other and capable of being manipulated to move relative to one another. In this manner, the endoscope 200 may be positioned in a close proximity or through the chest wall of the patient during navigating therethrough (e.g., through ribs of the patient). As can be appreciated, the elongated body 210 of the endoscope 200 may include any number of segments to aid maneuverability of the endoscope 200 within the body of the patient.

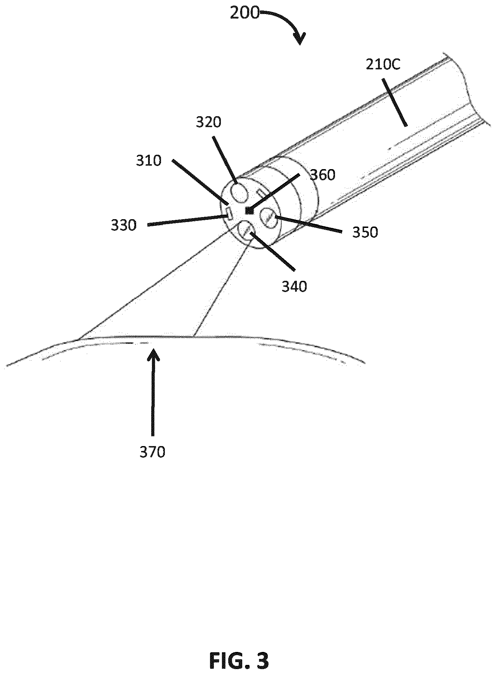

Referring to FIG. 3, the endoscope 200 may include an optical camera 320, a light source 330, a structured light (e.g., laser or infrared (IR)) projection source or structured light scanner ("scanner") 340, and a scan camera 350. Although generally illustrated as being disposed in a circular configuration about the distal surface 310 of the endoscope 200, the optical camera 320, the light source 330, the scanner 340, and the scan camera 350 may be disposed in any suitable configuration. The optical camera 320 may be a visual-light optical camera such as a charge-coupled device (CCD), complementary metal-oxide semiconductor (CMOS), N-type metal oxide semiconductor (NMOS), or any other such suitable camera. In one non-limiting example, the optical camera 320 is a CCD camera having a predetermined resolution (e.g., high definition (HD), full high definition (FHD), quad high definition (QHD), 4K, or 8K). The endoscope 200 may also have one or more electromagnetic (EM) sensors 360 disposed near the distal surface 310, or at any desired point along or within the endoscope 200, to facilitate location information of the one or more EM sensors 360, and any associated components of the endoscope 200, during EM navigation. The EM sensor 360 is configured to communicate with the electromagnetic tracking system.

The light source 330 may be a light emitting diode (LED) configured to emit white light. In embodiments, any LED configured to emit light having any one or more visible light frequencies may be used. The scanner 340 may be any structured light source, such as an LED, IR, or laser that is dispersed into a scan pattern (e.g., a line, mesh, dot matrix, etc.), by a rotating mirror or a beam splitter, which is not shown in FIG. 3. In embodiments, the scanner 340 may emit collimated light. The scan camera 350 may be a CCD camera capable of detecting the reflected light of the scan pattern from the target, although it is contemplated that the scan camera 350 may detect visible light, such as visible green light or the like, depending on the target being scanned. Specifically, visible green light contrasts with tissue having a red or pinkish hue, enabling the scan camera 350 to more easily identify the topography of the tissue or target. Likewise, visible blue light that is absorbed by hemoglobin may enable the system to detect vascular structures along with a vascular topology to act as additional reference points to be matched when aligning images captured by the optical camera 320. A digital filter (not explicitly shown) or filter having narrow band optical grating (not explicitly shown) may be used to inhibit extraneous visible light emitted from the scanner 340, thereby limiting the light exposure of the scan camera 350 within light emitted by the scanner 340 at a selected wavelength. In embodiments, the visible light is filtered from the image captured by the optical camera 320 and transmitted to the medical professional via the computing device 180 of FIG. 1 such that the image is clear and free from extraneous light patterns.

In embodiments, the scan camera 350 may be any thermographic camera known in the art, such as a ferroelectric, silicon microbolometer, or uncooled focal plane array (UFPA), or may be any other suitable visible light sensor such as a CCD, CMOS, NMOS, and the like, configured to sense light transmitted by the scanner 340.

In embodiments, the distal surface 310 may include a suitable transparent protective cover (not shown) capable of inhibiting fluids and/or other contaminants from coming into contact with the optical camera 320, the light source 330, the scanner 340, and the scan camera 350. Since the distance between the scanner 340 and the scan camera 350 relative to the optical camera 320 is fixed (e.g., the offset of the optical camera 320 relative to the scanner 340 and the scan camera 350), the images obtained by the optical camera 320 can more accurately obtained and, in embodiments, matched with pre-operative images.

In embodiments, the images captured by the optical camera 320 may be integrated with the images captured by the scan camera 350 to generate 3D scan data of the target or a surgical site of interest. The generated 3D scan data may include 3D structure (e.g., shape information in space) of the target. Since the 3D scan data is taken in close proximity of the target, the 3D scan data may include more detail information of the target than the 3D model, which is generated from magnetic resonance imaging, ultrasound, computer tomographic scan, positron emission tomography (PET), or the like, by the computing device 180 of FIG. 1. The 3D scan data, in embodiments, may be integrated with the 3D model of the patient to generate an intra-operation 3D model. Thus, the scanning system 100 may be able to supplement the 3D model of the patient during medical procedures with detail information of the target obtained from the 3D scan data. The scanning system 100 may track changes in the target by displaying the series of the 3D scan data, the images captured by the optical camera 320, or the series of the intra-operation 3D models.

In embodiments, the scanner 340 may be disposed on an outer surface of the third segment 210c. As can be appreciated, the location of the scanner 340 on the outer surface of the third segment 210c enables triangulation where the scanner 340 and the scan camera 350 are directed at an angle from the centerline of the third segment 210c (e.g., the scanner 340 and the scan camera 350 are disposed at an angle incident to a longitudinal axis defined by the third segment 210c).

The scan camera 350 has a field of view (FOV) 370, which is an area that the scan camera 350 can capture an image without significant distortion or deformation. The optical camera 320 also has a FOV. The FOV of the optical camera 320 may be greater than or equal to the FOV 370 of the scan camera 350. By aligning the FOVs of the optical camera 320 and the scan camera 350, images captured by the optical camera 320 may be aligned, compared, and/or integrated with images captured by the scan camera 350.

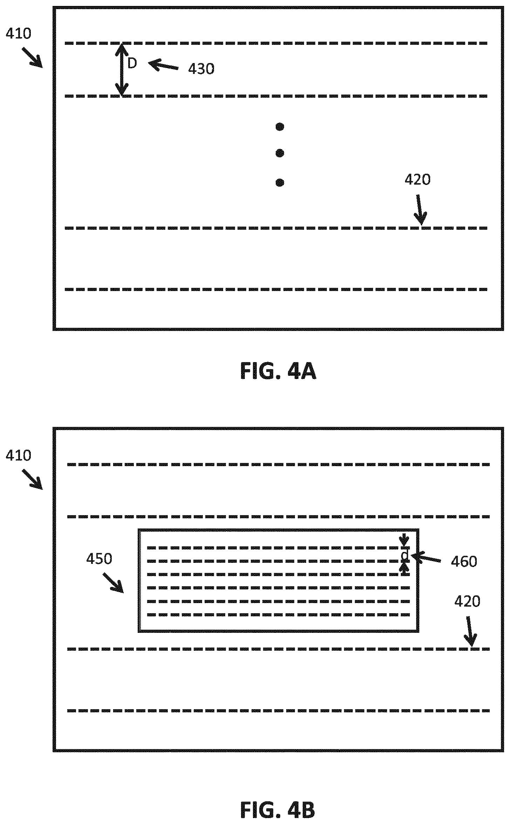

In embodiments, the shape of the FOVs of the optical camera 320 and the scan camera 350 may be rectangular, circular, or in any shape suitable for purposes used in the scanning system 100 of FIG. 1. For example, FIGS. 4A and 4B illustrate a rectangular shaped FOV 410. Further, FIGS. 4A and 4B illustrate two different scanning modes, coarse scanning and fine scanning, that the scanner 340 of FIG. 3 is able to perform, respectively.

Referring to FIGS. 4A and 4B, the scanner 340 initially performs the coarse scanning in the FOV 410. As shown in FIG. 4A, the scanner 340 emits the collimated light 420 in the FOV 410 in a coarse mode. The distance 430 between each collimated light is D. The time required to scan the FOV 410 in the coarse mode may be determined by the scanning speed ν of the scanner 340. For example, the time t₁ required for one scanning line may be calculated by dividing the width w of the FOV 410 by the scanning speed ν, i.e. t₁ = w/ν. Thus, the total scanning time T₁ for scanning the FOV 410 in the coarse mode is t₁ times the number n of scanning lines in the FOV 410, that is T₁ = ν ^{∗} n. The total scanning time T₁ for the coarse scanning may vary based on the shape or size of the FOV and the scanning speed.

The optical camera 320 captures a series of images along the passage of time with the scan camera 350 capturing images of the FOV 410 scanned by the scanner 340. The computing device 180 of FIG. 1 may compare the series of images captured by the optical camera 320 or a series of 3D scan data, which have been acquired by integrating the images captured by the optical camera 320 and the scan camera 350. When a change in the target is identified by the computing device 180, the area of the change may need to be further investigated by medical professionals. In such cases, the computing device 180 may automatically identify a focused area 450 within the FOV 410 of the scan camera 350.

In embodiments, the focused area 450 may be identified by comparing the 3D model of the patient and the series of images captured by the optical camera 320. When the area captured by the optical camera 320 is not included or not sufficiently shown in the 3D model, the area may be identified as the focused area 450.

In embodiments, the focused area 450 may be identified by comparing the series of the images captured by the scan camera 350. Two consecutive images are compared and, when a change is identified, the area of the change is identified as the focused area 450. In a case when the images are captured in a short time, two images captured in a predetermined period (e.g., 1 second, 2 seconds, etc.) may be compared. In embodiments, the series of 3D scan data may be compared to identify the focused area 450 in a similar manner.

In embodiments, the surgeon may manually identify the focused area 450 wherever further fine scanning is needed. A hand motion (e.g., pinching in or out) on a touch screen of the display may define the focused area 450. A joystick or foot peddle may be used to set boundaries of the focused area 450. Further, by following or tracking eye movements, the focused area 450 may be determined.

In embodiments, the focused area 450 may be automatically identified by the computing device 180 subject, in embodiments, to manual adjustment by the surgeon. The shape of the focused area 450 may be polygonal, e.g., rectangular or triangular, or rounded, e.g., circular. The shape of the focused area 450, however, may have an arbitrary shape based on the inputs from the surgeon and/or based on the area of the changes.

In embodiments, the focused area 450 may be identified as a portion of the FOV 410 of a selected size. For example, the focused area 450 may be a center portion of the FOV 410, a border portion of the FOV 410, a top, bottom, left, and/or right portion of the FOV 410, etc. The particular portion and size thereof may depend upon a user-input setting, a default setting, a direction of movement of the endoscope 200 (FIG. 3), or in any other suitable manner.

In embodiments, the focused area 450 may be identified based upon position(s) of surgical instrument(s) within the FOV 410. The position(s) of the surgical instrument(s) may be tracked using sensors, via visual identification using a camera, and/or via manual tagging by a surgeon. The focused area 450, in such embodiments, may be identified as an area surrounding the surgical instrument(s) that is centered on the surgical instrument(s), or may be defined as any other area relative to the surgical instrument(s) such as, for example, based upon a direction of movement of one or more of the surgical instruments, an area between two or more surgical instruments, etc.

Continuing with reference to FIG. 4A and 4B, after performing the coarse scanning and identifying the focused area 450, the scanner 340 performs a fine scanning over the focused area 450. As shown in FIG. 4B, the scanner 340 emits the scanning light having a smaller distance 460, d, between the consecutive scanning lights than the distance 430, D, in the coarse scanning. The total scanning time T₂ in the fine mode may be predetermined or preset. Thus, the smaller the focused area 450 is, the narrower the distance 460, d, is. Further, the smaller the focused area 450 is, the more detail information about the focused area 450 can be obtained from the scan image by the scan camera 350. The ratio of the fine scanning of the focused area 450 to the coarse scanning of the FOV 410 may be no less than one to one.

In embodiments, the coarse scanning and the fine scanning may be interleaved so that the image captured by the scan camera 350 may include two resolutions. The focused area 450 has a higher resolution than the other area in the FOV 410. Thus, the image captured by the scan camera 350 may provide more detail information of the focused area 450 than the other areas in the FOV 410.

In embodiments, scanning may be performed by a multiple scan lines (e.g., dual scan lines). By adding one or more scan lines, the total scanning time may be reduced by the factor of the number of lines. The noises or distortions made due to the multiple scan lines may be compensated by standard filters, such as nearest neighbor or mean value.

As described above, this image having two resolutions may be integrated with the image captured by the optical camera 320 to generate 3D scan data of the target. As the medical procedures or surgeries advance, the series of the 3D scan data may provide changes made to the target along the passage of the time.

Further, this 3D scan data may be integrated with the 3D model to generate an intra-operation 3D model. As the medical procedures or surgeries advance, the 3D model may be updated to reflect the changes made to the target.

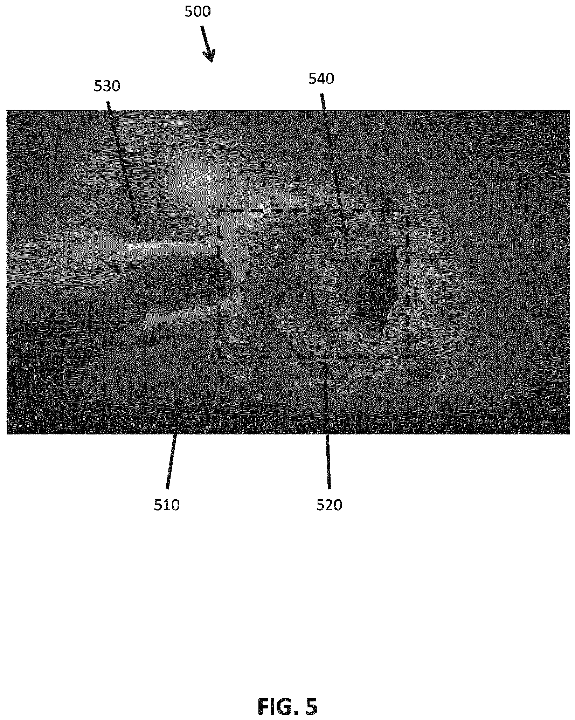

An image 500, as shown in FIG. 5, is a graphical example of images having two resolutions. The image 500 illustrates an image captured while navigating a luminal network of a lung. When a foreign object 540 is found in a bronchial tree during the navigation, coarse scanning is performed on a peripheral region 510 and fine scanning is performed in a central region 520, similarly as illustrated in FIGS. 4A and 4B. The central region 520 is a focused region captured from fine scanning and the peripheral region 510 is a region captured from the coarse scanning. As such, the central region 520 has a higher resolution than the peripheral region 510.

As shown in FIG. 5, the foreign object 540 is captured in the central region 520. Based on the detailed view of the foreign object 540, a medical instrument 530 (e.g., biopsy tool, ablator, stapler, end effectors, etc.) may be inserted and perform an operation on the foreign object 540. Further, the image 500 having two resolutions may be fused with the 3D model in a way so that the new 3D model may show more information than the previous 3D model.

Further, the image 500 or the new 3D model may be used later in time to check whether the foreign object 540 has been properly treated or removed based on a newly captured image having two resolutions.

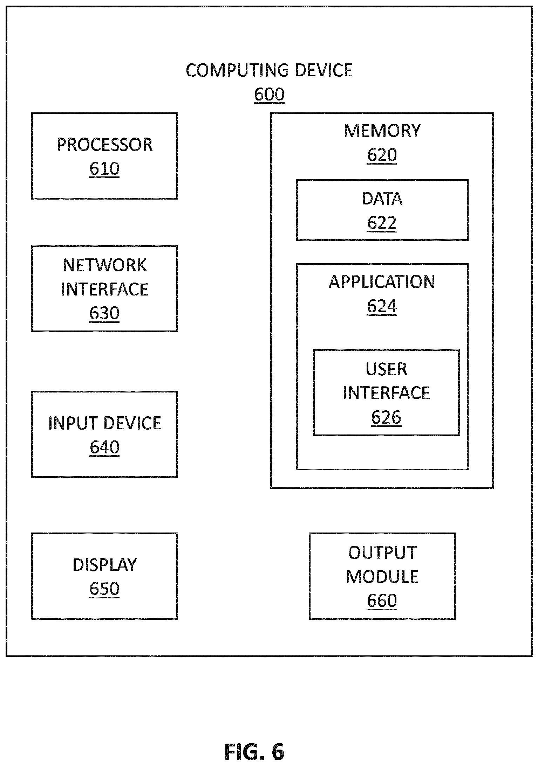

FIG. 6 shows a block diagram of a computing device 600, which can function as the computing device 180 of FIG. 1 or a separate computing device. The computing device 600 may include a processor 610, a memory 620, a network interface 630, an input device 640, a display 650, and/or an output module 660. Memory 620 may store applications 624 and/or image data 622. The application 624 may, when executed by the processor 610, execute sets of instructions to perform all functions of the scanning system 100 of FIG. 1 and/or of the endoscope of FIGS. 2-3, and cause the display 650 to display thereon a graphical user interface (GUI) 626. The application 624 may also provide the interface between the tracked position of EM sensor 360 of FIG. 3 and location information of the 3D model developed by the scanning system 100 of FIG. 1.

The processor 610 may be a general-purpose processor, a specialized graphics processing unit (GPU) configured to perform specific graphics processing tasks while freeing up the general-purpose processor to perform other tasks, and/or any number or combination of such processors.

The memory 620 may include any non-transitory computer-readable storage media for storing data and/or software that is executable by the processor 610 and which controls the operation of the computing device 600. In an aspect, the memory 620 may include one or more solid-state storage devices such as flash memory chips. Alternatively, or in addition to the one or more solid-state storage devices, the memory 620 may include one or more mass storage devices connected to the processor 610 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 610. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which can be used to store the desired information and which can be accessed by the computing device 600.

The network interface 630 may be configured to connect to a network such as a local area network (LAN) composed of a wired network and/or a wireless network, a wide area network (WAN), a wireless mobile network, a Bluetooth network, and/or the internet. The input device 640 may be any device by means of which a user may interact with the computing device 600, such as, for example, a mouse, keyboard, foot pedal, touch screen, and/or voice interface. The output module 660 may include any connectivity port or bus, such as, for example, parallel ports, serial ports, universal serial busses (USB), or any other similar connectivity port known to those skilled in the art. The display 650 may be touch-sensitive and/or voice-activated, enabling the display 650 to serve as both an input and output device.

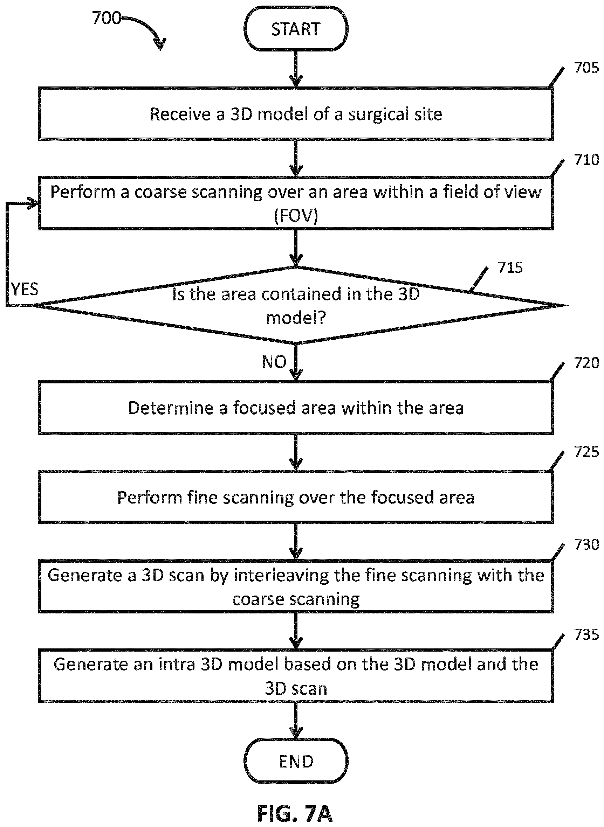

Provided by FIG. 7A is a flowchart illustrating a method 700 for updating a 3D model according to embodiments of the disclosure. The method 700 sets forth a process for updating the 3D model by performing coarse scanning and fine scanning, and starts by receiving a 3D model of a target or an internal organ of a patient in step 705. The 3D model may be generated from fluoroscopic or x-ray images or video of the patient by a scanning system.

An endoscope may approach the target based on the 3D model. When the endoscope is in close proximity to the target, a scanner of the endoscope may perform a coarse scanning over an area corresponding to the FOV of an image sensor (e.g., a camera) of the endoscope in step 710. In step 715, it is determined whether or not the area corresponding to the FOV is contained in the 3D model. When it is determined that the 3D model contains the FOV in step 715, the endoscope keeps performing the coarse scanning in step 710 until the FOV is not contained in the 3D model.

Since the 3D model has a lower resolution than the scan data from the coarse scanning, the 3D model may not have any information or may have a little information about the FOV. In this case, it is determined that the area of the FOV is not contained in the 3D model. Then, in step 720, the scanning system or a surgeon may determine or identify a focused area, which is not contained in the 3D model. The focused area is then scanned in the fine mode in step 725, meaning that the distance between consecutive scanning lines is smaller than the distance between consecutive scanning lines in the coarse mode. In embodiments, the total time for scanning the focused area in the fine mode may be predetermined. Thus, the distance between the consecutive scanning lines in the fine mode may be determined based on the predetermined total time and the size of the focused area.

In step 730, the coarse scanning and the fine scanning are interleaved to generate 3D scan data of the FOV. The 3D scan data has two different resolutions, meaning that the focused area has a higher resolution than that of the areas other than the focused area.

In step 735, the 3D scan data may be integrated at the corresponding location of the 3D model. This updated 3D model is an intra-operational or -procedural 3D model so that a series of intra 3D models may show progressive changes of the target. The method 700 may go back to step 710 and iterate steps 710-735 until the surgical procedure is complete.

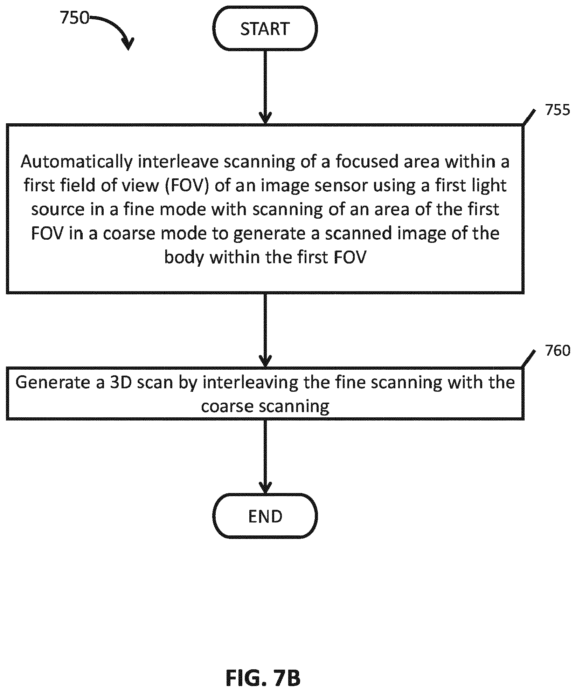

Provided by FIG. 7B is a flowchart illustrating a method 750 for generating a 3D scan according to embodiments of the disclosure. The method 750 starts by automatically interleaving scanning of a focused area within a first FOV of an image sensor using a first light source in a fine mode with scanning of an area of the first FOV in a coarse mode to generate a scanned image of the body within the first FOV in step 755. As such, the resolution of the focused area is higher than the resolution of the first FOV other than the focused area within the image captured from the interleaving scanning.

The 3D scan is generated by interleaving the fine mode scanning and the coarse mode scanning in step 760.

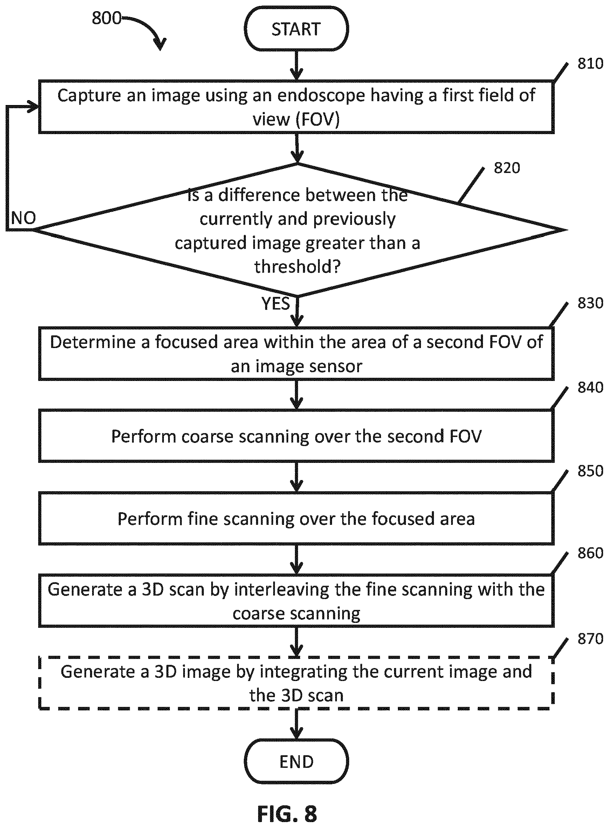

FIG. 8 shows a flowchart illustrating a method 800 for generating 3D scan data of a surgical site according to embodiments of the disclosure. The method 800 may be performed without receiving a 3D model. When an endoscope is inserted to navigate to a target inside of a patient, an optical camera of the endoscope captures an image of the target in step 810. The image captured by the optical camera shows a first FOV of the optical camera.

When a series of images are captured, it is determined whether or not a difference between the currently captured image and the previously captured image is greater than a threshold in step 820. The threshold may be predetermined as a minimum value indicating that there is a noticeable difference in the shape or structure of the target. For example, in a liver resection or dissection, when it is determined that the difference is not greater than the threshold, the difference may suggest that the liver is not sufficiently resected or dissected. In this case, the optical camera of the endoscope continues to capture images of the area corresponding to the first FOV in step 810 and to compare the difference with the threshold.

In a case when a difference is noticeable, the difference is determined to be greater than the threshold in step 820. In such case, a focused area is determined within a second FOV of a scan camera of the endoscope in step 830. In embodiments, the first FOV of the optical camera may be greater than or equal to the second FOV of the scan camera. The scan camera may be integrated in the endoscope. The scan camera may utilize a scanner, which emits structured or collimated light along a scanning pattern. The focused area is a portion of the second FOV.

In embodiments, steps 810 and 820 are skipped and the method begins at step 830 where a focused area is determined within a (second) FOV, e.g., wherein the focused area is selected in accordance with any of the embodiments detailed above.

The scanner may perform a scanning (coarse scanning) in a coarse mode within the second FOV in step 840 and perform a scanning (fine scanning) in a fine mode within the focused area in step 850.

In step 860, 3D scan data is generated by interleaving the coarse scanning and with fine scanning. The generated 3D scan data may be integrated with the currently captured image to generate a 3D image of the target in step 870. The method 800 may go back to 810 (or step 830) and perform steps 810-870 (or steps 830-870) until the surgical procedure is completed.

In embodiments, step 840 may be performed right after step 810 and before the determination in step 820. In this situation, the currently captured image and the 3D scan data obtained from step 840 may be integrated to each other to generate a 3D image. In step 820, the difference between the currently generated 3D image and the previously generated 3D image is compared with the threshold. When the difference is determined to be greater than the threshold in step 820, steps 850 and 860 are performed to generate an updated 3D scan data, and in step 870, a 3D image is generated by integrating the updated 3D scan data and the currently captured image. The series of 3D images may be displayed along the passage of time to show developments of changes in the target.

Detailed embodiments of the disclosure are disclosed herein. However, the disclosed embodiments are merely examples of the disclosure, which may be embodied in various forms and embodiments. Therefore, specific structural and functional details disclosed herein are not to be interpreted as limiting, but merely as a basis for the claims and as a representative basis for teaching one skilled in the art to variously employ the disclosure in virtually any appropriately detailed structure.

The described techniques in this disclosure may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

It should be understood that embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain embodiments of this disclosure are described as being performed by a single module or unit for purposes of clarity, it should be understood that the techniques of this disclosure may be performed by a combination of units or modules associated with, for example, a medical device.

The invention may be described by reference to the following numbered paragraphs:-
1. A method for generating a three-dimensional (3D) scan of a body inside of a patient, the method comprising:
   automatically interleaving scanning of a focused area within a first field of view (FOV) of an image sensor using a first light source in a fine mode with scanning of an area of the first FOV in a coarse mode to generate a scanned image of the body within the first FOV; and
   generating 3D scan data of the body within the first FOV based on the scanned image.
2. The method according to paragraph 1, wherein a distance between two consecutive scanning lines in the coarse mode is larger than a distance between two consecutive scanning lines in the fine mode.
3. The method according to paragraph 1, wherein a resolution outside of the focused area in the scanned image is lower than a resolution within the focused area in the scanned image.
4. The method according to paragraph 1, wherein a ratio of an area scanned in the coarse mode to an area scanned in the fine mode is greater than or equal to 1.
5. The method according to paragraph 1, further comprising:
   capturing a series of images of a portion of the body within a second FOV of an endoscope using a second light source.
6. The method according to paragraph 5, further comprising:
   calculating a difference between two of the series of images.
7. The method according to paragraph 6, wherein the focused area is received when the difference is greater than or equal to a predetermined threshold.
8. The method according to paragraph 6, wherein the focused area includes an area, where the difference resides in majority.
9. The method according to paragraph 5, wherein the second FOV of the endoscope is not less than the first FOV of the image sensor.
10. The method according to paragraph 5, further comprising receiving the focused area when an area overlapped by the second FOV and the first FOV is less than a predetermined area.
11. The method according to paragraph 5, further comprising automatically designating the focused area in which the first FOV and the second FOV do not overlap.
12. A three-dimensional (3D) scanner comprising:
   an image sensor having a first field of view (FOV) using a first light source and configured to generate a series of images of a body inside of a patient;
   a scan image sensor having a second FOV using a second light source, and configured to scan an area of the body within the second FOV and generate a scanned image; and
   a processor configured to control the scan image sensor to scan the area of the body within the second FOV in a coarse mode and within a focused area in a fine mode and to generate 3D scan data of the body within the second FOV based on the series of images and the scanned image,
   wherein the processor is further configured to control the scan image sensor to automatically interleave scanning the focused area in the fine mode with scanning the area within the second FOV in the coarse mode,
   wherein the focused area is located within the area of the body.
13. The 3D scanner according to paragraph 12, wherein the second light source emits infrared (IR) light.
14. The 3D scanner according to paragraph 12, wherein the first light source emits visible light.
15. The 3D scanner according to paragraph 12, wherein a distance between two consecutive scanning lines in the coarse mode is larger than a distance between two consecutive scanning lines in the fine mode.
16. The 3D scanner according to paragraph 12, wherein a resolution outside of the focused area in the scanned image is lower than a resolution within the focused area in the scanned image.
17. The 3D scanner according to paragraph 12, wherein the processor is further configured to calculate a difference between the series of images and the scanned image obtained in the coarse mode.
18. The 3D scanner according to paragraph 17, wherein the focused area is determined when the difference is greater than or equal to a predetermined threshold.
19. The 3D scanner according to paragraph 12, wherein the focused area is determined when an area overlapped by the second FOV and the first FOV is less than a predetermined area.
20. A method for generating a three-dimensional (3D) scan of a body inside of a patient, the method comprising:
   receiving a three-dimensional (3D) model of the body;
   determining if an area of a field of view (FOV) of a scan camera is contained in the 3D model;
   scanning the area of the FOV in a coarse mode when it is determined that the area of the FOV is contained in the 3D model;
   automatically interleaving scanning of a focused area within the FOV in a fine mode with scanning of the FOV in the coarse mode when it is determined that the area of the FOV is not contained in the 3D mode;
   generating a scanned image of the FOV by the scan camera; and
   generating an intra 3D model based on the 3D model and the scanned image by the image sensor.

## Claims

1. A method for generating a three-dimensional (3D) scan of a body inside of a patient, the method comprising:
automatically interleaving scanning of a focused area within a first field of view (FOV) of an image sensor using a first light source in a fine mode with scanning of an area of the first FOV in a coarse mode to generate a scanned image of the body within the first FOV; and
generating 3D scan data of the body within the first FOV based on the scanned image.

2. The method according to claim 1, wherein a distance between two consecutive scanning lines in the coarse mode is larger than a distance between two consecutive scanning lines in the fine mode.

3. The method according to claim 1 or claim 2, wherein a resolution outside of the focused area in the scanned image is lower than a resolution within the focused area in the scanned image.

4. The method according to any preceding claim, wherein a ratio of an area scanned in the coarse mode to an area scanned in the fine mode is greater than or equal to 1.

5. The method according to any preceding claim, further comprising:
capturing a series of images of a portion of the body within a second FOV of an endoscope using a second light source; preferably further comprising:
calculating a difference between two of the series of images.

6. The method according to claim 5, wherein the focused area is received when the difference is greater than or equal to a predetermined threshold; and/or wherein the focused area includes an area, where the difference resides in majority.

7. The method according to claim 5 or claim 6, wherein the second FOV of the endoscope is not less than the first FOV of the image sensor.

8. The method according to any of claims 5 to 7, further comprising receiving the focused area when an area overlapped by the second FOV and the first FOV is less than a predetermined area; preferably further comprising automatically designating the focused area in which the first FOV and the second FOV do not overlap.

9. A three-dimensional (3D) scanner comprising:
an image sensor having a first field of view (FOV) using a first light source and configured to generate a series of images of a body inside of a patient;
a scan image sensor having a second FOV using a second light source, and configured to scan an area of the body within the second FOV and generate a scanned image; and
a processor configured to control the scan image sensor to scan the area of the body within the second FOV in a coarse mode and within a focused area in a fine mode and to generate 3D scan data of the body within the second FOV based on the series of images and the scanned image,
wherein the processor is further configured to control the scan image sensor to automatically interleave scanning the focused area in the fine mode with scanning the area within the second FOV in the coarse mode,
wherein the focused area is located within the area of the body.

10. The 3D scanner according to claim 9, wherein the second light source emits infrared (IR) light.

11. The 3D scanner according to claim 9 or claim 10, wherein the first light source emits visible light; and/or wherein a distance between two consecutive scanning lines in the coarse mode is larger than a distance between two consecutive scanning lines in the fine mode.

12. The 3D scanner according to any of claims 9 to 11, wherein a resolution outside of the focused area in the scanned image is lower than a resolution within the focused area in the scanned image.

13. The 3D scanner according to any of claims 9 to 12, wherein the processor is further configured to calculate a difference between the series of images and the scanned image obtained in the coarse mode; preferably wherein the focused area is determined when the difference is greater than or equal to a predetermined threshold.

14. The 3D scanner according to any of claims 9 to 13, wherein the focused area is determined when an area overlapped by the second FOV and the first FOV is less than a predetermined area.

15. A method for generating a three-dimensional (3D) scan of a body inside of a patient, the method comprising:
receiving a three-dimensional (3D) model of the body;
determining if an area of a field of view (FOV) of a scan camera is contained in the 3D model;
scanning the area of the FOV in a coarse mode when it is determined that the area of the FOV is contained in the 3D model;
automatically interleaving scanning of a focused area within the FOV in a fine mode with scanning of the FOV in the coarse mode when it is determined that the area of the FOV is not contained in the 3D mode;
generating a scanned image of the FOV by the scan camera; and
generating an intra 3D model based on the 3D model and the scanned image by the image sensor.
